# EUROPEAN PATENT SPECIFICATION

(11) **EP 2 875 807 B1**
(45) Date of publication and mention of the grant of the patent: **24.03.2021**
(21) Application number: 14193816.7
(22) Date of filing: 19.11.2014
(51) Int. Cl.: A61K 9/20, A61K 9/28, A61K 31/785

(54) **TABLET FORMULATION OF COLESEVELAM**
TABLETTENFORMULIERUNG VON COLESEVELAM
FORMULATION DE COMPRIMÉ DE COLESEVELAM

(30) Priority: 20.11.2013 TR 201313512
(43) Date of publication of application: 27.05.2015
(73) Proprietor: Sanovel Ilac Sanayi ve Ticaret A.S., 34460 Istanbul (TR)
(72) Inventor: Türkyilmaz, Ali, 34460 Istanbul (TR); Yildirim, Ediz, 34460 Istanbul (TR); Karabulut, Tutku Ceren, 34460 Istanbul (TR); Hatirnaz, Zekiye Basak, 34460 Istanbul (TR)
(74) Representative: Sevinç, Erkan

(56) References cited:
- US-A1- 2010 330 175
- US-A1- 2011 159 087
- US-A1- 2012 321 711
- BRIAN R. ROHRS ET AL: "Particle size limits to meet USP content uniformity criteria for tablets and capsules", JOURNAL OF PHARMACEUTICAL SCIENCES, vol. 95, no. 5, January 2006 (2006-01), pages 1049-1059, XP055077685, ISSN: 0022-3549, DOI: 10.1002/jps.20587
- LIEBERMAN H A ET AL: "PHARMACEUTICAL DOSAGE FORMS, Tablets", 1 January 1980 (1980-01-01), XP003028605,

## Description

### Technical Field

The present invention concerns a solid oral pharmaceutical composition of colesevelam useful as cholesterol lowering agent.

### Background of the Invention

Colesevelam, disclosed in US patent 5,693,675, is a modified crosslinked polyallylamine polymer known as 1-chloro-2,3-epoxypropane, [6-(allylamino)-hexyl]trimethylammonium chloride and N-allyldecylamine, hydrochloride and is useful in methods of lowering the serum cholesterol level of a patient. It is currently available in dosage forms suitable for oral administration including tablet forms under the commercial name Welchol® in US and Cholestogel® in Europe.

In prior art, tablet forms of colesevelam are firstly disclosed in US Patent 6,733,780. The patent describes a tablet core comprising at least about 95% by weight of an aliphatic amine polymer. Desired tablets of the patent are prepared by using direct compression method wherein the moisture content of polymer is adjusted from 5 to 7% by weight. Another method for producing tablets of polyallylamine polymers is disclosed in the patent EP2309977 wherein the method comprises slug compression step of polyallylamine having a density greater than 1.24 g/cm3. Both methods have an object to overcome hardness and disintegration problems occurred by high dose of amine polymers. However, there still exist compressibility problems when high dose tablet formulation of colesevelam is obtained by simple manufacturing process.

US application 20100008988 discloses pharmaceutical composition comprising less than about 95% by weight of an aliphatic amine polymer and PCT application WO2011135591A1 discloses a pharmaceutical composition comprising less than 75% by weight of aliphatic amine polymer. However, none of them provide a tablet formulation wherein compressibility properties are enhanced even if colesevelam dose is reduced. Therefore, there still exists a need for new compressible tablet formulations of colesevelam useful in methods of lowering the serum cholesterol level of a patient.

US 2011/159087 A1 discloses pharmaceutical compositions comprising wet granulated bile acid sequestrants, such as colesevelam and their process of preparation. The formulation comprises a coated tablet comprising a core which comprises 50-85wt. % colesevelam; wherein particle size of colesevelam is less than 150 µm and d90 value is less than 100 µm.

US 2010/330175 A1 discloses a tablet composition comprising colesevelam hydrochloride in combination with at least one pharmaceutically acceptable excipient and water. In particular, tablet formulation comprises 76wt.% colesevelam, wherein the particles of colesevelam have a particle size of less than 75 µm.

US 2012/321711 A1 relates to alkylated amine polymer, particularly colesevelam hydrochloride compositions which minimize the overall amount of the material administered to a patient.

The present invention provides a pharmaceutical tablet composition which avoids the afore-mentioned disadvantages of the high loaded tablet formulations of the prior art and have advantages over them.

The main object of the present invention is to obtain a pharmaceutical tablet composition wherein compressibility properties are enhanced.

Another object of the present invention is to provide a pharmaceutical tablet composition comprising colesevelam and at least one pharmaceutically acceptable excipient wherein amount of colesevelam and excipient is adjusted as not showing flowability and content uniformity problems during the manufacturing process.

Another object of the present invention is to provide a pharmaceutical tablet composition of colesevelam wherein agglomeration problem which occurs because of the hygroscopic property of colesevelam is minimalized.

Yet another object of the present invention is to provide a pharmaceutical tablet composition of colesevelam wherein a significantly improved pharmaceutical stability, as well as the excellent long-term stability is shown.

Yet another object of the present invention is to provide a pharmaceutical tablet composition comprising a core containing colesevelam and at least one coating layer containing at least one pharmaceutically acceptable excipient.

### Detailed Description of the Invention

In order to achieve the aforementioned objects, the present invention discloses a novel pharmaceutical tablet composition according to claim 1, for the oral administration of colesevelam as the active ingredient comprising one or more pharmaceutically acceptable excipient.

The term "colesevelam" as used herein refers to colesevelam as well as pharmaceutically acceptable salts, enantiomers, hydrates, solvates and mixture thereof. The term also includes all polymorphic forms, whether crystalline or amorphous. According to the present invention, the preferred form of colesevelam is colesevelam hydrochloride.

According to an embodiment of the present invention, said pharmaceutical tablet composition contains 250 to 1000 mg of colesevelam and preferably 500 to 750 mg of colesevelam or pharmaceutically acceptable salts thereof.

Said pharmaceutical tablet composition comprises a core and at least one coating layer covering the core, wherein the core contains colesevelam or pharmaceutically acceptable salt thereof.

Said pharmaceutical tablet composition comprises a core containing colesevelam as an active agent and at least one pharmaceutically acceptable excipient, according to claim 1. The present invention also provides that the core contains preferably uncoated colesevelam particles.

As it is known, colesevelam is a hygroscopic water-swelling amine polymer. Now, it is found that when colesevelam percentage by weight of the composition is high; its flowability is affected negatively. Therefore, the present invention provides that core of the tablet comprises 70 to 93%, 76 to 92%, 75 to 87% or 75 to 85% of colesevelam by weight of the core or pharmaceutically acceptable salt thereof. The percentage of colesevelam is adjusted as its flowability property is enhanced while agglomeration which occurs because of hygroscopicity of colesevelam is minimized. However, reducing percentage of colesevelam influences uniformity of content in the pharmaceutical tablet composition of the present invention. Lack of content uniformity in the present tablet formulation causes insufficienct drug content. To minimize this problem, we have developed particles of colesevelam having d₉₀ value less than 150 µm which provide pharmaceutical composition having content uniformity as well as flowable.

In an embodiment of the present invention, particle size distribution of colesevelam is measured by Malvern® Particle Size Analyser which is conventional method using laser diffraction. When measuring the particle size distribution by Malvern®, an appropriate amount of colesevelam is put into a plate preferably with water. From here, colesevelam particles are flowing down to measuring chamber and its size measured by laser diffraction.

The term "d₉₀" as used in the present invention means that the size at which %90 by volume of the particles is finer.

According to the present invention, said pharmaceutical tablet composition comprises a core and at least one coating layer wherein the core comprises 70 to 93%, 76 to 92%, 75 to 87% or 75 to 85% of colesevelam by weight of the core and wherein particles of colesevelam have d₉₀ value less than 150 µm.

According to an embodiment of the present invention, said pharmaceutical tablet composition comprises a core containing colesevelam or pharmaceutically acceptable salt thereof and at least one pharmaceutically acceptable excipient and at least one coating layer containing at least one pharmaceutically acceptable excipient; wherein excipients can be selected from the group comprising diluents, binders, lubricants, glidants, coating agents, plasticizers, wetting agents and disintegrants.

According to the present invention, microcrystalline cellulose is used as diluent.

According to a preferred embodiment of the present invention, said pharmaceutical tablet composition comprises a core containing at least one diluent which is microcrystalline cellulose, wherein its content is from 1% to 14% by weight of the core, preferably is present from 2% to 7% by weight of the core.

According to the present invention, said pharmaceutical tablet composition comprises a core containing colesevelam or pharmaceutically acceptable salt thereof and microcrystalline cellulose as diluent wherein the concentration ratio of colesevelam to microcrystalline cellulose, is in the range of between 15:1 and 1:0.5. Right concentration of colesevelam and diluent provide a better flowability and compaction property in the pharmaceutical tablet formulation.

Suitable glidants may include but not limited to colloidal silicon dioxide, talc, aluminium silicate and mixtures thereof; preferably the glidant is colloidal silicon dioxide.

According to a preferred embodiment of the present invention, said pharmaceutical tablet composition comprises a core containing at least one glidant wherein its content is from 1% to 13% by weight of the core, preferably is present from 2% to 8% by weight of the core.

According to another preferred embodiment of the present invention, the concentration ratio of colesevelam to glidant, preferably colloidal silicon dioxide, is in the range of between 25:1 and 1:2; and preferably 20:1 and 1:4.

According to an aspect, a pharmaceutical tablet composition is described which comprises a core containing 1% to 14% of microcrystalline cellulose, 1% to 13% of colloidal silicon dioxide and 70 to 93% of colesevelam by weight of the core wherein particles of colesevelam have d₉₀ value less than 250 µm or preferably less than 150 µm and wherein the concentration ratio of colesevelam to microcrystalline cellulose is in the range of between 20:1 and 1:0.05.

Suitable lubricants may include but not limited to magnesium strearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, sodium stearyl fumarate, sodium lauryl sulphate and mixtures thereof; preferably the lubricant is magnesium stearate and sodium lauryl sulfate. Lubricant content is from 0.01% to 10% by weight of total composition.

Suitable binders may include but not limited to polyvinylpyrrolidone, polyethylene glycol, polyvinyl alcohol, starch, pregelatinized starch, glucose, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, methacrylate polymers, xanthan gum and mixtures thereof. Binder content is from 1% to 20%, preferably from 2% to 10% by weight of total composition.

Suitable disintegrants may include but not limited to cross-linked polyvinil pyrrolidone (crospovidone), cross-linked carboxymethyl cellulose (croscarmellose sodium), low-substituted hydroxypropyl cellulose, pregelatinized starch, sodium carboxymethyl cellulose, calcium carboxymethyl cellulose, sodium alginate, corn starch, sodium starch glycolate and mixtures thereof.

Suitable coating agents may include but not limited to hydroxypropyl methylcellulose, hydroxypropyl cellulose, hydroxyethyl cellulose, methylcellulose, polyvinylpyrrolidone and mixture thereof; preferably hydroxypropyl methylcellulose.

According to a preferred embodiment of the present invention, said pharmaceutical tablet composition comprises at least one coating layer comprising at least one coating agent wherein its content is from 1% to 20% by weight of the total composition, preferably is present from 3% to 15% by weight of the total composition.

Suitable plasticizer may include but not limited to acetylated monoglyceride, diacetylated monoglyceride, triacetin, triethyl citrate, citric acid esters, polyethylene glycol, cetyl alcohol, polysorbates, phtalic acid esters and mixtures thereof; and preferably diacetylated monoglycerid or acetylated monoglyceride.

According to a preferred embodiment of the present invention, said pharmaceutical tablet composition comprises at least one coating layer comprising at least one plasticizer wherein its content is from 1% to 10% by weight of the total composition, preferably is present from 2% to 7% by weight of the total composition.

Further described is a method of producing tablets of colesevelam or pharmaceutically acceptable salt thereof, preferably direct compression, comprising the steps
i) mixing colesevelam or pharmaceutically acceptable salt thereof with at least one pharmaceutically acceptable excipient selecting from diluents, glidants and lubricants; wherein the resulting mixture comprises 70 to 93% by weight, 76 to 92% by weight, 75 to 87% by weight or 75 to 85% by weight of colesevelam or pharmaceutically acceptable salt thereof;
ii) optionally milling the mixture obtained by step i)
iii) compressing the mixture obtained by step i) or ii);
iii) coating the resulting mixture with a film layer comprising at least one pharmaceutically acceptable excipient selecting from coating agents and plasticizers.

According to an embodiment of the present invention, said pharmaceutical tablet composition of colesevelam can be administered alone or in combination with at least one other drug, preferably cholesterol absorption inhibitors as ezetimibe; statins as simvastatin, atorvastatin, rosuvastatin and lovastatin; fibrates (PPAR agonists) as fenofibrate, bezafibrate or cinofibrate; biguanides as metformin; glitazones as pioglitazone, rivoglitazone, rosiglitazone and troglitazone; sulfonylureas as glibenclamide, nateglinide, repaglinide, glimepiride and gliclazide; DPPIV inhibitors as gemigliptin, linagliptin, saxagliptin, sitagliptin and vildagliptin.

According to an embodiment of the present invention, said pharmaceutical tablet composition of colesevelam can be used in a method for removing bile acid salts from a patient, for reducing blood cholesterol, for treating atherosclerosis and for treating hypercholesterolemia in a patient.

## Claims

1. A pharmaceutical tablet composition comprising a core and at least one coating layer wherein the core comprises 70 to 93% of colesevelam by weight of the core and microcrystalline cellulose as a diluent; the concentration ratio of colesevelam to microcrystalline cellulose is in the range of between 15:1 and 1:0,5 and the particles of colesevelam have d₉₀ value less than 150 µm.

2. The pharmaceutical tablet composition according to claim 1, wherein the core comprises 76 to 92% of colesevelam by weight of the core.

3. The pharmaceutical tablet composition according to claim 1, wherein the core comprises 75 to 87% of colesevelam by weight of the core.

4. The pharmaceutical tablet composition according to claim 1, wherein the core comprises 75 to 85% of colesevelam by weight of the core.

5. The pharmaceutical tablet composition according to claim 1, wherein the core comprises at least one pharmaceutically acceptable glidant.

6. The pharmaceutical tablet composition according to claims 5 , wherein glidants are selected from the group comprising colloidal silicon dioxide, talc, aluminium silicate and mixtures thereof.

7. The pharmaceutical tablet composition according to claims 6, wherein glidant is colloidal silicon dioxide.

## Patentansprüche

1. Pharmazeutische Tablettenzusammensetzung, die einen Kern und zumindest eine Überzugsschicht aufweist, wobei der Kern 70 bis 93 Gew.-% Colesevelam bezogen auf den Kern, sowie mikrokristalline Zellulose als ein Verdünnungsmittel aufweist; wobei das Konzentrationsverhältnis von Colesevenam zu mikrokristalliner Zellulose in dem Bereich von zwischen 15:1 und 1:0,5 liegt, und wobei die Partikel von Colesevenam einen d₉₀-Wert von kleiner als 150 µm aufweisen.

2. Pharmazeutische Tablettenzusammensetzung gemäß Anspruch 1, wobei der Kern 76 bis 92 Gew.-% Colesevelam bezogen auf den Kern aufweist.

3. Pharmazeutische Tablettenzusammensetzung gemäß Anspruch 1, wobei der Kern 75 bis 87 Gew.-% Colesevelam bezogen auf den Kern aufweist.

4. Pharmazeutische Tablettenzusammensetzung gemäß Anspruch 1, wobei der Kern 75 bis 85 Gew.-% Colesevelam bezogen auf den Kern aufweist.

5. Pharmazeutische Tablettenzusammensetzung gemäß Anspruch 1, wobei der Kern zumindest ein pharmazeutisch akzeptables Gleitmittel aufweist.

6. Pharmazeutische Tablettenzusammensetzung gemäß Anspruch 5, wobei die Gleitmittel ausgewählt sind aus der Gruppe aufweisend kolloidales Siliziumdioxid, Talk, Aluminiumsilikat, und Mischungen davon.

7. Pharmazeutische Tablettenzusammensetzung gemäß Anspruch 6, wobei das Gleitmittel kolloidales Siliziumdioxid ist.

## Revendications

1. Composition de comprimé pharmaceutique comprenant un coeur et au moins une couche d'enrobage, dans laquelle le coeur comprend 70 à 93 % de colesevelam en poids du coeur et de la cellulose microcristalline en tant que diluant, le rapport de concentration du colesevelam à la cellulose microcristalline étant dans la plage d'entre 15 : 1 et 1 : 0,5 et les particules de colesevelam ayant une valeur d₉₀ inférieure à 150 µm.

2. Composition de comprimé pharmaceutique selon la revendication 1, dans laquelle le coeur comprend 76 à 92 % de colesevelam en poids du coeur.

3. Composition de comprimé pharmaceutique selon la revendication 1, dans laquelle le coeur comprend 75 à 87 % de colesevelam en poids du coeur.

4. Composition de comprimé pharmaceutique selon la revendication 1, dans laquelle le coeur comprend 75 à 85 % de colesevelam en poids du coeur.

5. Composition de comprimé pharmaceutique selon la revendication 1, dans laquelle le coeur comprend au moins un agent glissant pharmaceutiquement acceptable.

6. Composition de comprimé pharmaceutique selon la revendication 5, dans laquelle les agents glissants sont choisis dans le groupe comprenant le dioxyde de silicium colloïdal, le talc, le silicate d'aluminium et les mélanges de ceux-ci.

7. Composition de comprimé pharmaceutique selon la revendication 6, dans laquelle l'agent glissant est le dioxyde de silicium colloïdal.
